(19) Europäisches Patentamt
European Patent Office
Office européen des brevets

(11) Publication number: 0 002 051
B1

# (12) EUROPEAN PATENT SPECIFICATION

(45) Date of publication of patent specification: 03.02.88

(51) Int. Cl.[4]: A 61 K 31/46

(21) Application number: 78101367.7

(22) Date of filing: 14.11.78

## (54) Use of butoxybenzylhyoscyamine bromide in pharmaceutical compositions against deafness and tinnitus.

(30) Priority: 16.11.77 JP 136620/77

(43) Date of publication of application:
30.05.79 Bulletin 79/11

(45) Publication of the grant of the patent:
03.02.88 Bulletin 88/05

(84) Designated Contracting States:
DE FR GB NL

(56) References cited:
FR-M- 24
US-A-3 696 110

Chemical Abstracts vol. 83, no. 532, page 52 (1975)

UNLISTED DRUGS, vol. 24, no. 11, 1972, 166h

UNLISTED DRUGS, vol. 26, no. 4, 1974, 53f

(73) Proprietor: Eisai Co., Ltd.
6-10, Koishikawa 4-chome Bunkyo-ku
Tokyo 112 (JP)

(72) Inventor: Yuasa, Ryo
4-6-6, Dainohara Sendai-shi
Miyagi (JP)

(74) Representative: Eitle, Werner, Dipl.-Ing.
Arabellastrasse 4
D-8000 München 81 (DE)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to the use of butoxybenzylhyoscyamine bromide for the preparing of a pharmaceutical composition against deafness and tinnitus.

Causes of deafness and tinnitus have not been completely elucidated and a satisfactory curative method has not been established. For remedy of deafness and tinnitus, there have been employed vitamins such as vitamins A, $B_1$, $B_2$, $B_6$ and $B_{12}$, pantothenic acid, chondroitin sulfate, ATP and vasolidators. However, satisfactory curative effects can hardly be attained by these drugs.

In FR—A—24M an anti-tinnitus effect of N-allyl hyoscyamine is mentioned. No further details are given in this respect.

Under such background, investigations have been made with a view to developing an effective medicament for remedy of deafness and tinnitus and it has surprisingly been found that butoxybenzylhyoscyamine bromide, whose anti-spasmodic activity is known, is effective in pharmaceutical compositions against deafness, especially perceptive deafness, and tinnitus.

Most of the caues of perceptive deafness are unknown and this disease is accompanied by irreversible changes, whereas the causes of conductive deafness are substantially known. The treatment of perceptive deafness could hardly be expected by medicinal therapy. However, it has been found that with a pharmaceutical composition even perceptive deafness can be effectively cured. Accordingly, the present invention makes great contributions to cure of deafness.

The general name of butoxybenzylhyoscyamine bromide that is used in the present invention is butropium bromide, and this compound is a synthetic antispasmodic for digestive canals which has been used for alleviating spasmodic pains in gastritis, enteritis, gastric ulcer, duodenal ulcer, cholelithiasis and cholecystitis. This compound has the following structural formula:

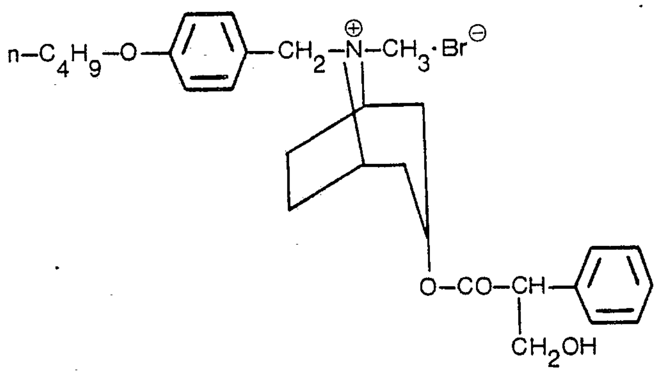

This compound is a white crystal or crystalline powder having a melting point of about 166°C, cf for instance C.A. *83*, 532 p (1975).

It is a primary object of the present invention to provide a novel use of the above compound for the preparation of a drug against deafness, tinnitus and impaired hearing.

The amount administered of butoxybenzylhyoscyamine bromide can vary depending on the degree of deafness or tinnitus, but when it is administered to adults, the amount administered is oridinarily about 1 to about 200 mg per day.

The agent may be administered orally or by injection, and the administration form is not particularly limitative. The agent may be applied in the form of a powder, a granule, a tablet, a capsule, an injection or a suppository. The agent may be formed into such drug by using pharmaceutical carriers customarily used in this field.

Toxicity of butoxybenzylhyoscyamine bromide in detail:

(A) The acute toxicity, $LD_{50}$ (mg/kg), is as shown in table 1.

TABLE 1

| Animal | Sex | Administration | | | |
|---|---|---|---|---|---|
| | | Oral | hyperdermic | intramuscular | intravenous |
| rat | ♂ | >1600 | >800 | >480 | 21 |
| rat | ♀ | >1600 | >800 | >480 | 21 |
| mouse | ♂ | 1920 | 370 | 285 | 8.0 |
| mouse | ♀ | 1600 | 570 | 320 | 6.4 |
| rabbit | ♂ | — | — | — | 6.8 |

(B) The compound was administered in an amount of 3.2, 8.0 or 20 mg/kg per day to male and female rats continuously during 25 weeks to determine the chronic toxicity. In the group of rats in which the daily administered amount was 20 mg/kg, there were observed such changes as inactive motion, mydriasis, control of increase of the body weight, storage of a small quantity of abdominal dropsy and formation of false membranes on the liver and spleen.

However, no substantial change was observed in any organ, and particular changes or disorders were not observed at all at tests of blood and urine.

In order to clarify the effects of the present invention, results of clinical tests will now be described in detail below with reference to drawings in which:

Figures 1 to 6 show audiograms obtained in Cases 1 to 6. The dates affixed to the audiograms indicate the measurement dates. (Japanese calendar).

(I) Object:

Among patients who came into the hospital during the period of from September 1976 to September 1977 while complaining of deafness or tinnitus, those meeting the following conditions were chosen.

(1) Patents suffering from perceptive deafness where the pure-tone region was clinically considered to be fixed, or patients suffering from everlasting tinnitus though hearing loss was not observed.

(2) Patients who had previously received one or other medicinal treatment but with no improvement.

(3) Adults who were generally healthy and did not suffer from other complications, especially glaucoma and benign prostatic hypertrophy.

Butoxybenzylhyoscyamine bromide was administered to 74 patients meeting the above conditions.

(II) Method:

A mixture of 4 mg of butoxybenzylhyoscyamine bromide with 20% glucose was intravenously injected at first, and then, two capsules, each containing 5 mg of butoxybenzylhyoscyamine bromide, was administered 3-times per day (i.e. 6 capsules were administered per day). The administration of capsules was conducted for one week.

(III) Measurement Items:

(a) The standard audiometry (125—8000 Hz, air and bone condition) was carried out before the injection.

(b) The same test was conducted 30 minutes after the injection. In patients complaining of tinnitus, the change of the degree of tinnitus was examined.

(c) The audiometry was carried out on the 7th day from the start of the oral administration. If the patients felt changes of hearing power or tinnitus during this period, the audiometry was appropriately conducted.

(d) During the oral administration period of 7 days, disease conditions such as (1) hearing power (2) tinnitus, (3) other ear symptoms and (4) side effects were recorded on examination cards.

(IV) Results

1. Change of hearing power just after injection (30 minutes):

With respect to 50 patients, the audiometry was carried out just after the intravenous injection of butoxybenzylhyoscyamine bromide (30 minutes after the injection) to obtain results shown in table 2. Sounds of 250 Hz, 1000 Hz and 8000 Hz were chosen as typical instances of sounds of the low-tone, medium-tone and high-tone regions, respectively, and changes of the hearing power to these sounds were examined. The change within a range of ±5 dB was judged as "no change" and the change exceeding 5 dB was judged as "improvement" or "worsening".

TABLE 2

| | dB | 250 Hz | | 1000 Hz | | 8000 Hz | |
|---|---|---|---|---|---|---|---|
| worsening | −10 | 8% | | 2% | | 2% | |
| | − 5 | 8 | | 12 | | 6 | |
| no change | 0 | 36 | 74% | 54 | 90% | 54 | 86% |
| | + 5 | 30 | | 24 | | 26 | |
| | +10 | 12 | | 2 | | 8 | |
| improvement | +15 | 6 | 18% | 4 | 8% | 4 | 12% |
| | +20 | 0 | | 2 | | 0 | |

As will be apparent from the results shown in table 2, the highest improvement was 20 dB (1000 Hz) but in some cases, the improvement was up to 15 dB. The improvement was higher in the order of the low-tone region (18%), the high-tone region (12%) and the medium-tone region (8%) and the average improvement of the hearing power after the intravenous injection was about 13%.

2. Improvement of Tinnitus:
With respect to 41 patients complaining of tinnitus, the change of degree of tinnitus was examined after one weeks's oral administration to obtain results shown below in table 3.

TABLE 3

| Rank | Change | Contents of Change | Number patients |
|---|---|---|---|
| 1 | remarkably effective | tinnitus disappeared condition was highly improved | 5 |
| 2 | effective | tinnitus did not disappear but was relieved | 10 |
| 3 | ineffective | no change | 24 |
| 4 | worsening | tinnitus was worsened | 2 |

As will be apparent from the results shown in table 3, the agent was effective in 15 patients (36%), though the agent of the present invention was ineffective in 24 patients (59%) or the agent worsened the state in 2 patients (5%).

3. Change of hearing power after administration:
Changes of the hearing power were examined during a period of 1 year and three months at longest after one week's administration. With respect to cases where high improving effects were attained, the clinical history will be described.

Case 1 (male, 35 years old)
On September 4, 1976, a patient suffering from perceptive deafness was measured for his hearing power by the audio-gram, the result of the audiogram is indicated in the curve 1 of Fig. 1. Six capsules, each containing 5 mg of butoxybenzylhyoscyamine bromide, were administered to the patient per day. The change of the hearing power was traced to obtain the results shown in the audiograms of Fig. 1. As will be apparent from Fig. 2, the improvement was observed in the low-tone region after the administration and the hearing loss was reduced since then. As is obvious from the audiograms 2 and 3 obtained on September 6 and 16, 1976, respectively, the hearing power was improved even after stopping of the administration.

Case 2 (male, 54 years old)
On August 17, 1976, the patient was first examined. He complained that deafness had occurred in the left ear about 10 days before. Perceptive deafness of flat hearing loss of about 30 dB was observed in the left ear and the recruitment phenomenon was positive. C.P. was observed on the left side in the static sense test. Accordingly, the sellate ganglion block (hereinafter referred to as S.G.B.") was conducted and simultaneously, vitamin $B_1$ and the like were administered. However, the hearing power was gradually reduced and when one month had passed from occurrence of deafness, hearing loss was about 50 dB as shown in the audiogram 1 of Fig. 2. During this period, however, the patient did not feel dizzy. On September 4, 1976, oral administration of butoxybenzylhyoscyamine bromide was started while conducting S.G.B., and when the administration was continued for one week, the hearing power was returned to the level equal to the level at the first examination, and the hearing loss was gradually reduced even after stopping of the administration. After about 1 month, namely on October 7, 1976, it was found that the hearing loss was reduced to 10 dB. Tinnitus disappeared with the improvement of the hearing power.

Case 3 (male, 21 years old)
On March 29, 1977, deafness suddenly occurred in the right ear and the patient complained of dizziness. The audiogram was flat and perceptive deafness of hearing loss of 50 to 60 dB was observed. Vasodilators and vitamins $B_1$, $B_6$ and $B_{12}$ were administered for treatment of sudden deafness, but no substantial improvement of the hearing power was attained. During this period, dizziness disappeared. At the time of first examination, spontaneous nystagmus toward the affected ear was observed, but it immediately disappeared. Accordingly, the case was diagnosed as fixed sudden deafness and intravenous

injection of butoxybenzylhyoscyamine bromide was conducted 6-times on April 16, 19, 26 and 38 and May 9, 1977, respectively. The reduction of the hearing power, which had been considered to be permanent, was remarkably improved in the low-tone region (see audiograms 1 to 4 of Fig. 3).

Case 4 (female, 56 years old)

The patient had suffered from deafness since 1952—1953, but did not receive any medical treatment. Since about 10 days before the visit to the hospital, pains had been felt in both ears. No substantial change was observed on the drum membranes, and the case was diagnosed as perceptive deafness of both ears showing abrupt hearing loss in the high-tone region (see audiogram 1 of Fig. 4).

From the clinical history, the case was diagnosed as fixed perceptive deafness, and intravenous injection of butoxybenzylhyoscyamine bromide was conducted once and the same agent was then orally administered for 1 week. Afte one week's oral administration, the hearing power was gradually improved and when 3 weeks had passed, the hearing power was restored by 20 dB (2 KHz) at maximum and by about 10 dB on the average as seen from the audiogram 2 of Fig. 4. Then, the oral administration was conducted again for 1 weeks. At the point of termination of the oral administration (May 6, 1977), it was found that the hearing power was further improved in the low-tone region. Then, the hearing power was periodically examined, and on June 27, 1977, the hearing power was restored to a level shown in the audiogram 4 of Fig. 4. Thus, it has been confirmed that the agent of the present invention is effective even for the treatment of long-standing deafness, when the patient had given up the expectation of treatment.

Case 5 (male, 56 years old)

The patient complained of perceptive deafness of the left ear, and vitamins $B_1$, $B_6$ and $B_{12}$ and vasodilators were administered from December 28, 1976 to January 19, 1977. The hearing power was not improved but rather worsened. From January 19, 1977, butoxybenzylhyoscyamine bromide was orally administered for 3 weeks in an amount of 6 capsules per day. The hearing power was gradually recovered but was not returned to the normal level (see audiograms 1 to 3 of Fig. 5).

Case 6 (male 67 years old)

In this case, the change of the hearing power could not be traced, but it was found that the hearing loss was extremely reduced. 30 Minutes after intravenous injection of butoxybenzylhyoscyamine bromide, the audiogram 2 of Fig. 6 was obtained.

Examples for the use of butoxybenzylhyoscyamine bromide in the formulation of drugs against deafness will now be described, though the present invention by no means limited by these examples.

Preparation Example 1 (capsules)

| | |
|---|---|
| Butoxybenzylhyoscyamine bromide | 5.0 g |
| Crystalline cellulose | 50.0 g |
| Corn starch | 25.0 g |
| Lactose | 20.0 g |
| Total | 100.0 g |

Capsules were prepared from the foregoing ingredients according to customary procedures.

Preparation Example 2 (injection)

| | |
|---|---|
| Butoxybenzylhyoscyamine bromide | 4 g |
| Glucose | 50 g |
| Distilled water for injection | appropriate amount |
| Total | 1000 ml |

Injection ampoules, each containing 4 mg of butoxybenzylhyoscyamine bromide in 1 ml of the liquid, were prepared from the above ingredients according to customary procedures.

**Claim**

Use of butoxybenzylhyoscyamine bromide for the preparation of pharmaceutical compositions against deafness and tinnitus.

**Patentanspruch**

Verwendung von Butoxybenzylhyoscyaminbromid zur Herstellung einer pharmazeutisch aktiven Zubereitung gegen Taubheit und Tinnitus.

**Revendication**

Utilisation du bromure de butoxybenzylhyoscyamine pour la préparation de compositions pharmaceutiques contre la surdité et les acouphènes.

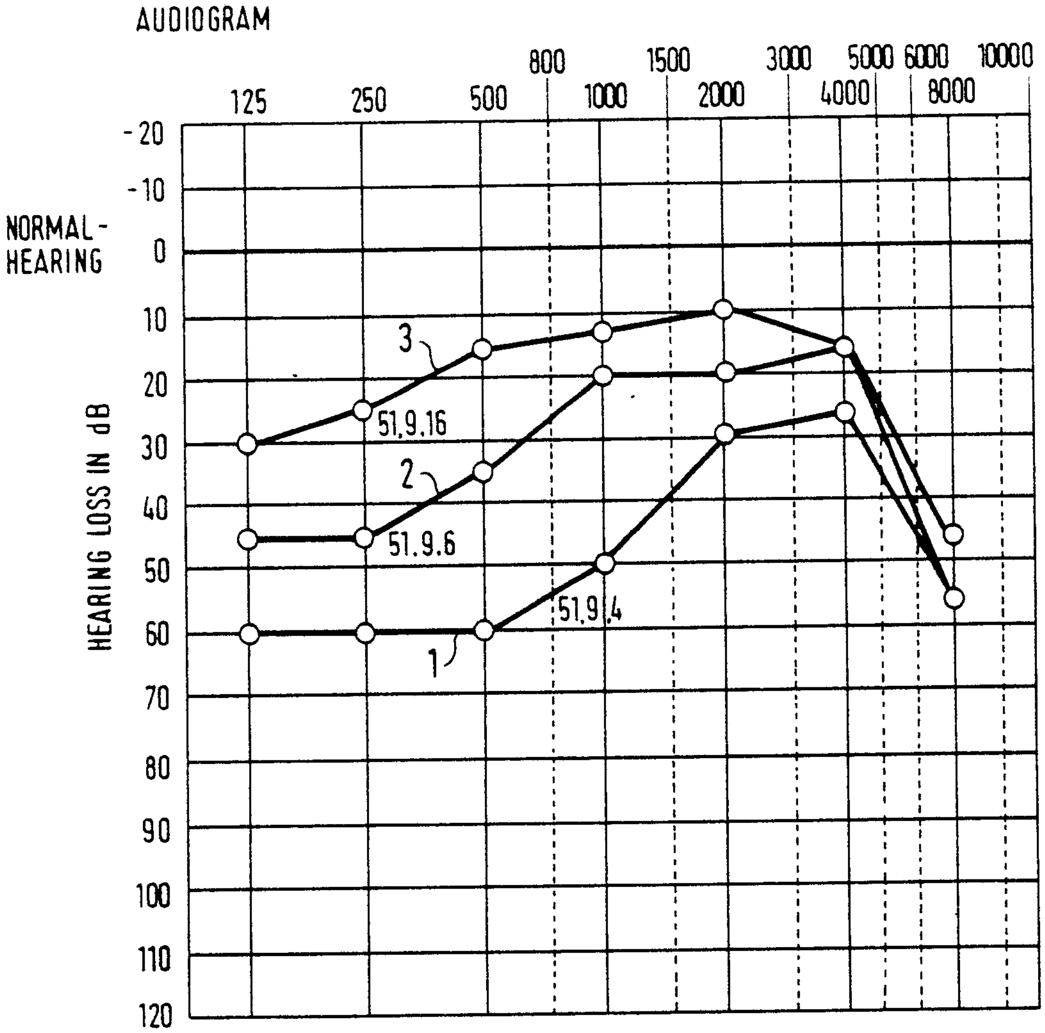
AUDIOGRAM
800
1500
3000
5000
6000
10000
125
250
500
1000
2000
4000
8000
-20
-10
NORMAL-
HEARING
0
10
20
30
40
50
60
70
80
90
100
110
120
HEARING LOSS IN dB
3
51,9.16
2
51.9.6
51,9.4
1

F I G. 1

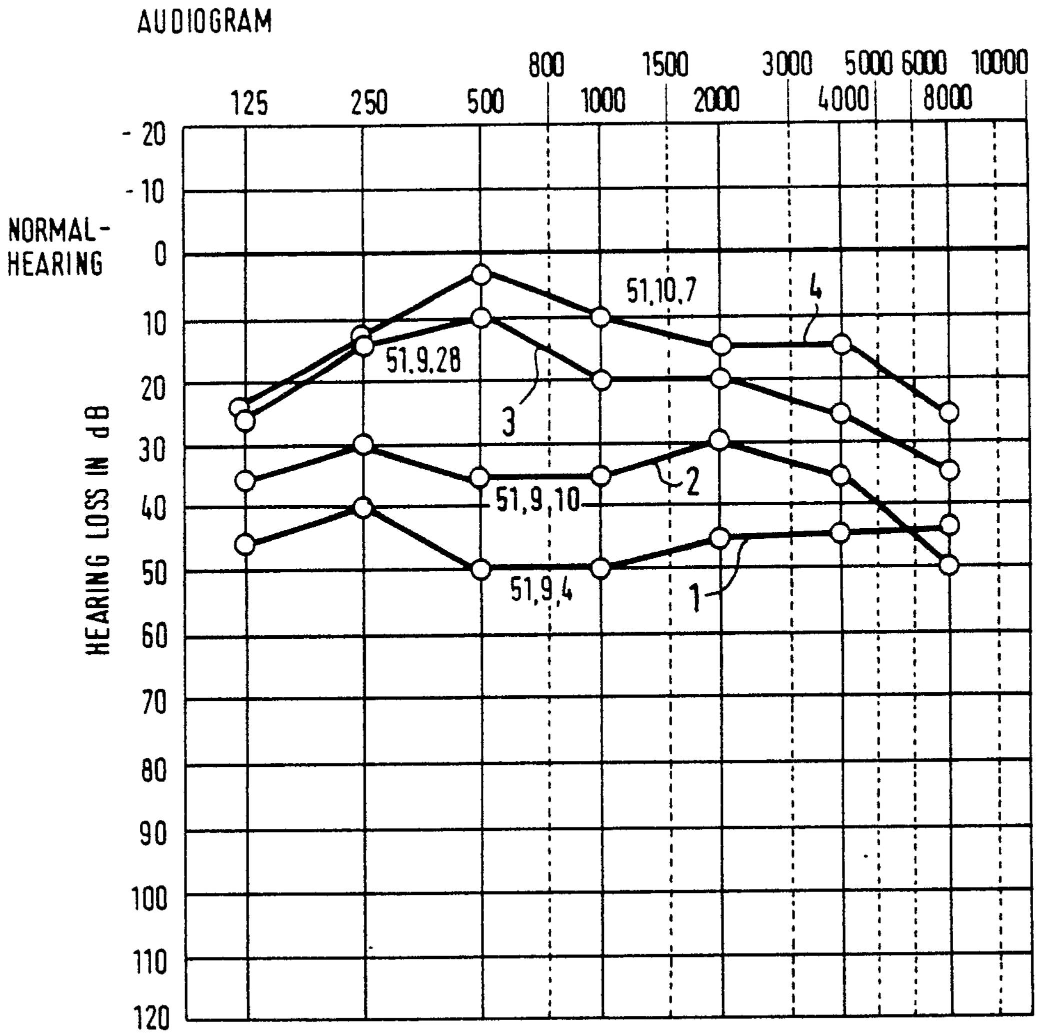
AUDIOGRAM
800
1500
3000
5000
6000
10000
125
250
500
1000
2000
4000
8000
- 20
- 10
NORMAL-HEARING
0
10
20
30
40
50
60
70
80
90
100
110
120
HEARING LOSS IN dB
51,10,7
4
51,9,28
3
2
51,9,10
51,9,4
1

FIG.2

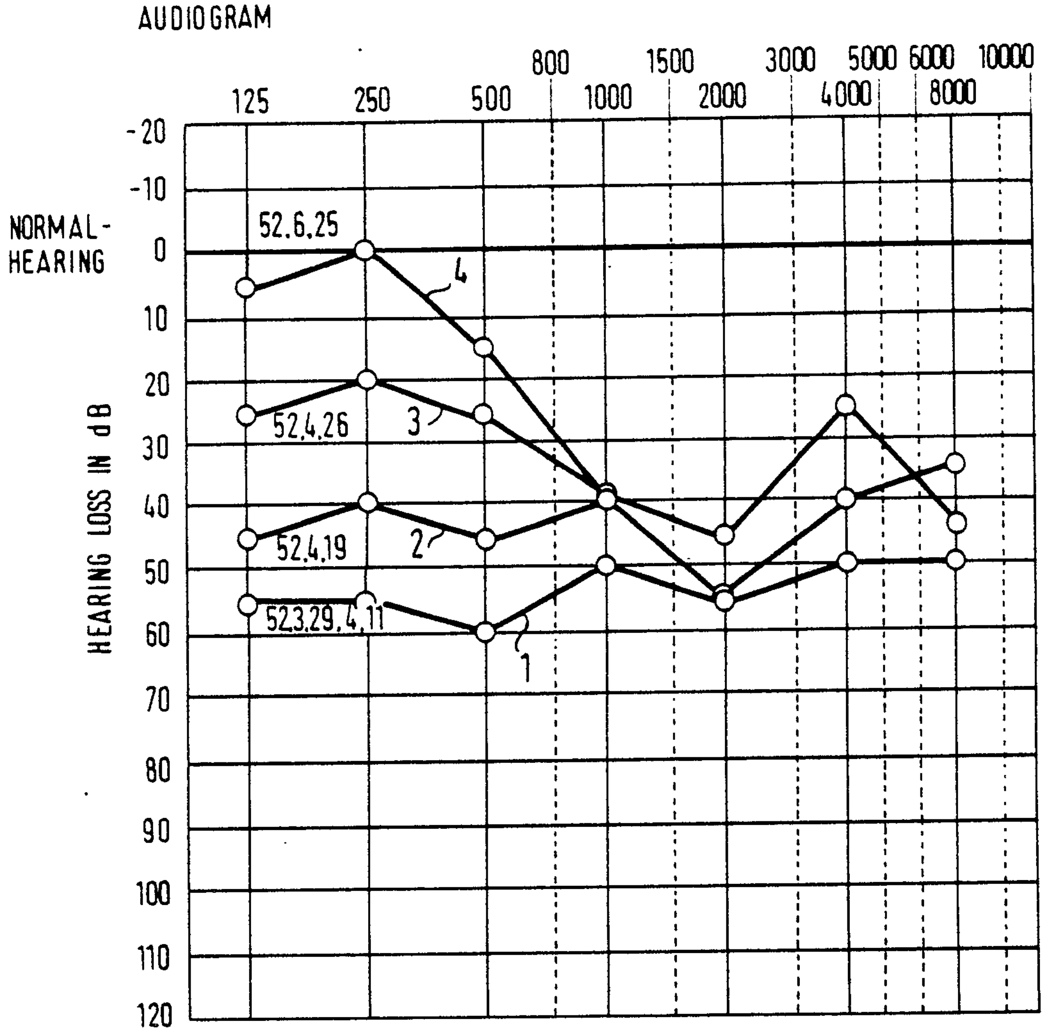
AUDIOGRAM
125
250
500
800
1000
1500
2000
3000
4000
5000
6000
8000
10000
-20
-10
0
10
20
30
40
50
60
70
80
90
100
110
120
NORMAL-
HEARING
HEARING LOSS IN dB
52.6.25
52.4.26
52.4.19
52.3.29, 4.11
1
2
3
4

FIG.3

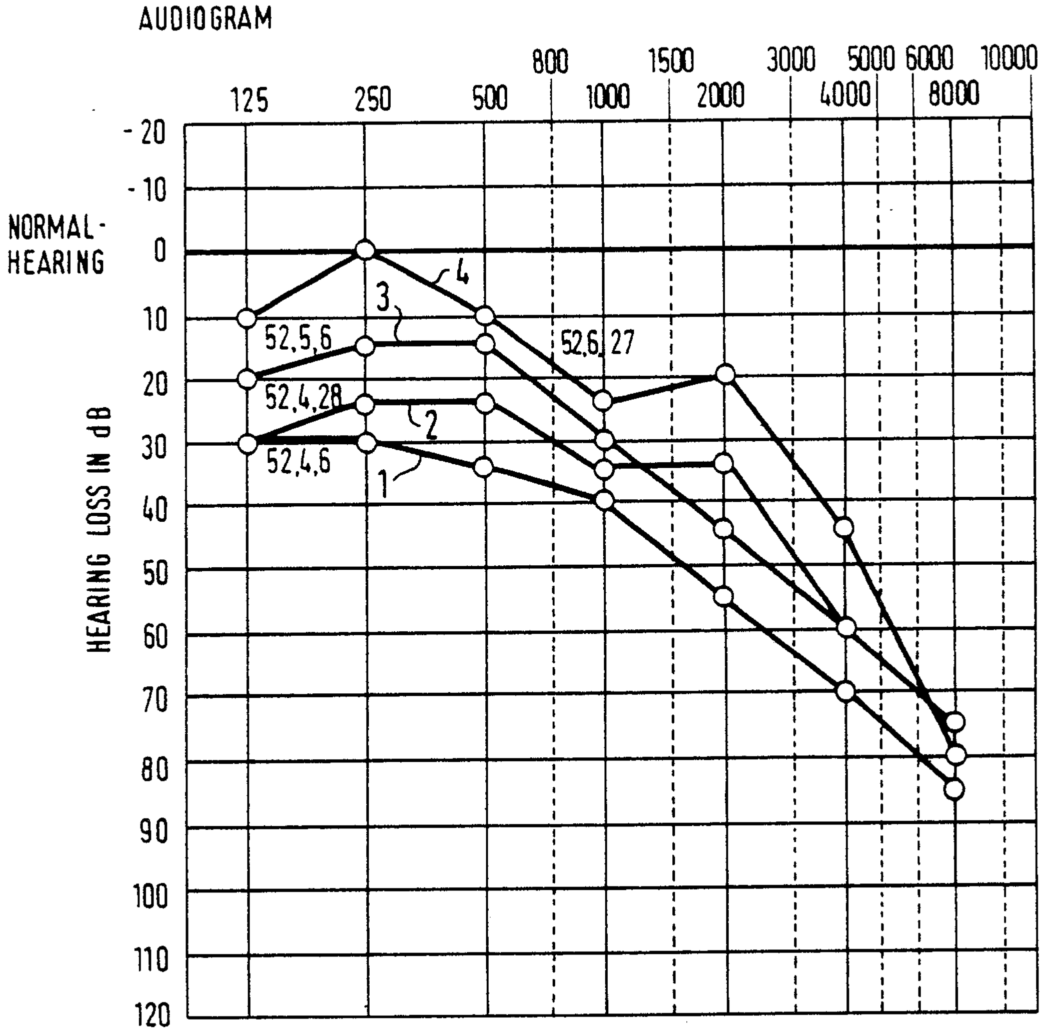
AUDIOGRAM
800
1500
3000
5000
6000
10000
125
250
500
1000
2000
4000
8000
-20
-10
NORMAL-
HEARING
0
10
20
30
40
50
60
70
80
90
100
110
120
HEARING LOSS IN dB
52.5.6
52.4.28
52.4.6
52.6.27
1
2
3
4

FIG.4

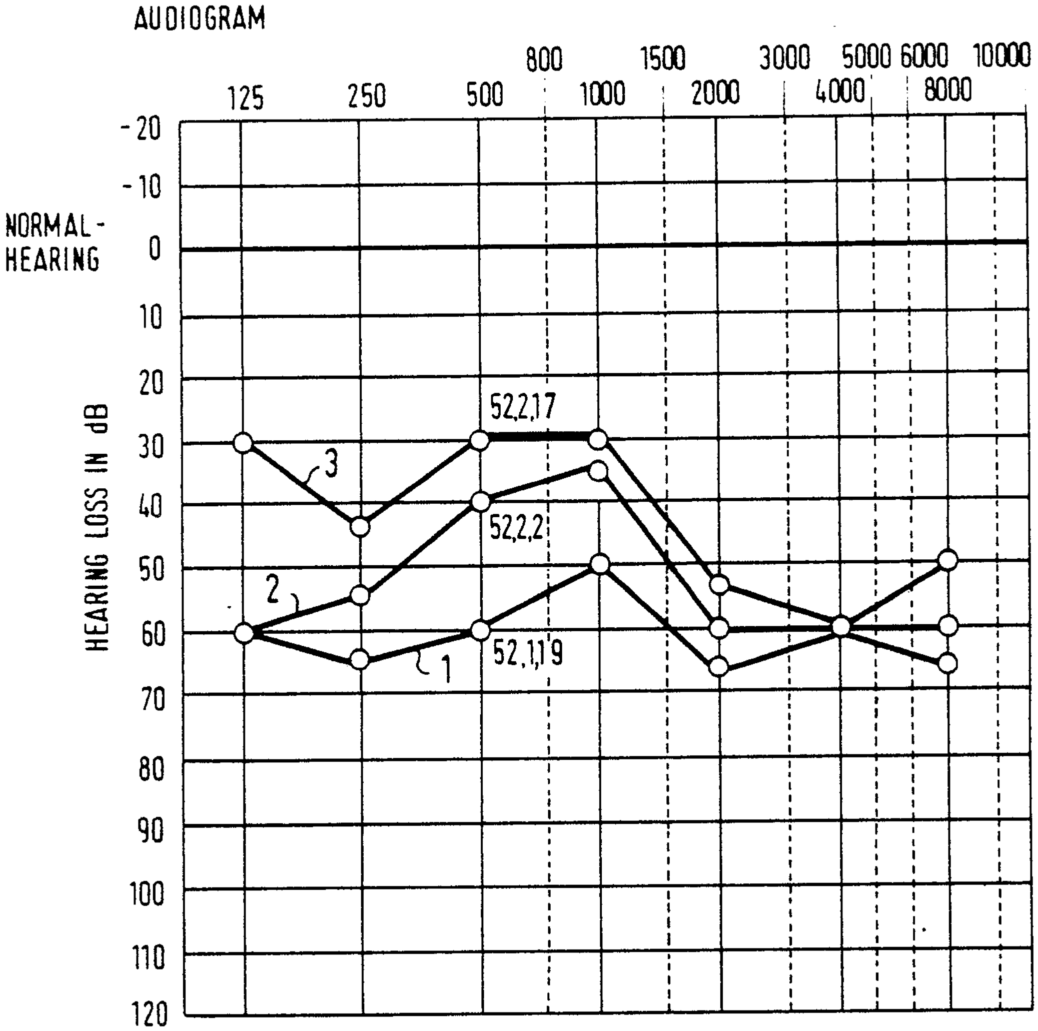
AUDIOGRAM
125
250
500
800
1000
1500
2000
3000
4000
5000
6000
8000
10000
-20
-10
NORMAL-HEARING
0
10
20
30
40
50
60
70
80
90
100
110
120
HEARING LOSS IN dB
52,2,17
52,2,2
52,1,19
1
2
3

FIG.5

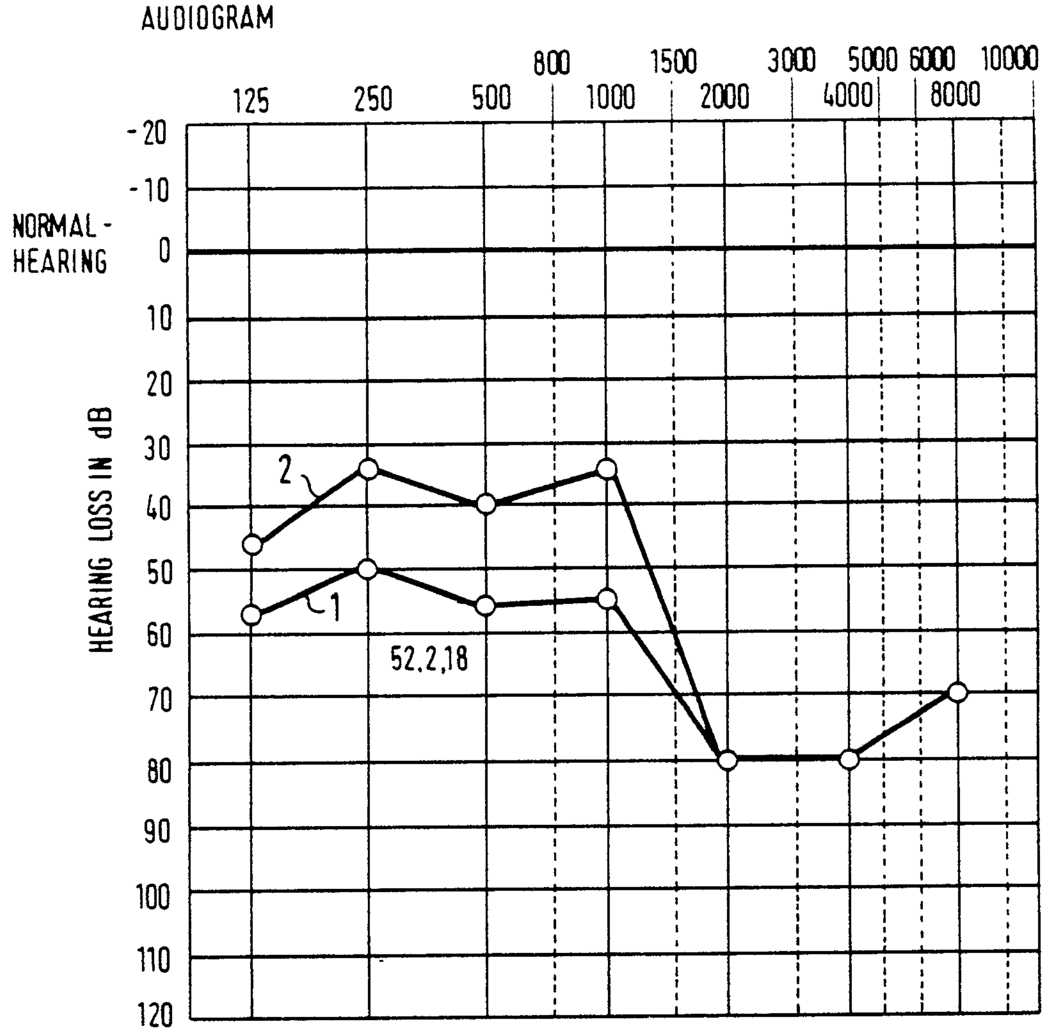
AUDIOGRAM
800
1500
3000
5000
6000
10000
125
250
500
1000
2000
4000
8000
-20
-10
NORMAL-HEARING
0
10
20
30
40
50
60
70
80
90
100
110
120
HEARING LOSS IN dB
2
1
52,2,18

FIG. 6